## Europäisches Patentamt

## European Patent Office

## Office européen des brevets

(19)

(11) Veröffentlichungsnummer: **0 164 656**
**B1**

(12) **EUROPÄISCHE PATENTSCHRIFT**

(45) Veröffentlichungstag der Patentschrift: 19.09.90

(21) Anmeldenummer: **85106657.1**

(22) Anmeldetag: **30.05.85**

(51) Int. Cl.⁵: **C 12 P 19/18, A 23 L 1/22**

(54) Gluco-Oligosaccharid-Gemisch und Verfahren zu seiner Herstellung.

(30) Priorität: **15.06.84 DE 3422247**

(43) Veröffentlichungstag der Anmeldung:
**18.12.85 Patentblatt 85/51**

(45) Bekanntmachung des Hinweises auf die
Patenterteilung:
**19.09.90 Patentblatt 90/38**

(84) Benannte Vertragsstaaten:
**AT BE CH DE FR GB IT LI NL**

(56) Entgegenhaltungen:
**CARBOHYDRATE RESEARCH, Band 108, 1982,
Seiten 279-283, Elsevier Scientific Publishing
Co., Amsterdam, NL; K.L.SMILEY et al.: "A
simplified method for preparing linear isomaltooligosaccharides"**

**CARBOHYDRATE RESEARCH, Band 121, 1983,
Seiten 279-286, Elsevier Science Publishers B.V.,
Amsterdam, NL; J.F.ROBYT et al.: "Relative,
quantitative effects of acceptors in the reaction
of leuconostoc mesenteroides B-512F
detransucrase"**

(73) Patentinhaber: **PFEIFER & LANGEN
Linnicher Strasse 48
D-5000 Köln 41 (DE)**

(72) Erfinder: **Schwengers, Dieter, Dr.
Jussenhovener Strasse 37
D-4047 Dormagen 1 (DE)**

(74) Vertreter: **Eggert, Hans-Gunther, Dr.
Räderscheidtstrasse 1
D-5000 Köln 41 (DE)**

**EP 0 164 656 B1**

**Beschreibung**

Kalorienarme Süßungsmittel oder solche aus einem Kohlehydratträger und üblichen Süßstoffen, wie Saccharin, Zyklamat oder Acesulfam-K, sind bekannt.

Die Erfindung betrifft derartige Süßungsmittel bzw. Träger in Form eines iso-Malto-Oligosaccharid-Gemisches mit bis zu 30, insbesondere 10 bis 20, Anhydroglucoseeinheiten. Diese Oligosaccharide fielen bisher nur als Abfallprodukt bei der Herstellung klinischer Dextrane durch Säurehydrolyse von hochmolekularem nativen Dextran an, hatten aber eine sehr breite Molekulargewichtsverteilung, die von Glucose bis zu Molgewichten von etwa 50000 reichte.

Der Erfindung liegt die Aufgabe zugrunde, iso-Malto-Oligosaccharid-Gemische mit bis zu 30, insbesondere 10 bis 20, Anhydroglucoseeinheiten in einer jeweils gewünschten engen Molekulargewichtsverteilung zu synthetisieren.

Diese Aufgabe wird erfindungsgemäß dadurch gelöst, daß man einer wässrigen Lösung eines aus D-Glucoseeinheiten aufgebauten Mono- oder Disaccharids, die mehr als 200 mMol des Saccharids pro 1000 U $\alpha(1\rightarrow6)$-D-Glucosyltransferase enthält, bei 265 bis 310 K und einem pH-Wert von 4,5 bis 8,0 eine wässrige Saccharoselösung in einer solchen Menge zugibt, daß das molare Verhältnis von Saccharose zu Glucose 2,0 bis 5,0 beträgt.

Das Reaktionsgemisch wird vorzugsweise bei 290 bis 300 K und in einem pH-Wert-Bereich von 5 bis 6,5 gehalten. Beide Parameter haben einen Einfluß auf die Struktur der entstehenden Produkte.

Als $\alpha(1\rightarrow6)$-D-Glucosyltransferase nach der Klassifizierung der "Enzyme Commission" bezeichnet man Enzyme, die die D-Glucopyranosylgruppe der Saccharose auf geeignete Akzeptoren übertragen. Ein solches extracelluläres Enzym ist Dextransucrase (E.C. 2.4.1.5.), das von gewissen Bakterienarten der Lactobacilli-Familie, z.B. Leuconostoc mesenteroides, insbesondere dem Stamm B-512, Leuconostoc dextranicum, Streptococcus und Lactobacillus gebildet wird. Zur Herstellung von Dextran dient in erster Linie Saccharose als Akzeptor und wirkt als Ketteninitiator für eine Kettenpolymerisation, bei der durch fortlaufende Übertragung von D-Glucopyranosyl-Gruppen aus der Saccharose auf die wachsende Kette das Polysaccharid Dextran mit Molmassen von mehreren Millionen gebildet wird, während gleichzeitig für jedes Molekül umgesetzter Saccharose ein Molekül Fructose frei wird.

Wenn man bei dieser Reaktion andere Mono-, Di- oder Tri-saccharide als Akzeptor einsetzt, werden auf Kosten des Dextrans in geringem Umfang Oligosaccharide gebildet. Bei Einsatz von Glucose als Akzeptor entstehen etwa 78% Dextran und als Nebenprodukt etwa 9% Oligosaccharide (IM-3 bis IM-12). Mit Maltose als Akzeptor werden niedere Gluco-oligosaccharide mit bis zu 6 Anhydroglucose-Einheiten neben nativem Dextran erhalten (Robyt und Eklund, Carbohydrate Research 121 (1983), 279—286). Typischerweise werden die Oligosaccharide mit steigendem Polymerisationsgrad in abnehmender Menge gebildet.

Unter den erfindungsgemäßen Reaktionsbedingungen gelingt es, die Übertragung von Glucosyl-Gruppen aus der Saccharose auf aus D-Glucose-Einheiten aufgebaute Mono- und Disaccharide, wie Glucose oder Maltose, so zu lenken, daß kein natives Dextran mehr entsteht, sondern in hoher Ausbeute die Gluco-oligosaccharide mit bis zu 30, insbesondere 10 bis 20 Anhydroglucoseeinheiten, aufgebaut werden. Überraschend dabei ist, daß die Glucooligosaccharide mit steigendem Polymerisationsgrad nicht mehr in abnehmender Menge gebildet werden, sondern daß in Abhängigkeit von der umgesetzten Saccharosemenge Gluco-Oligosaccharide eines bestimmten Polymerisationsgrades bevorzugt gebildet werden.

Bei dem erfindungsgemäßen Verfahren empfiehlt es sich, zur Erzielung hoher Ausbeuten der gewünschten Oligosaccharide die wässrige Lösung von Saccharose mit einer solchen Geschwindigkeit kontinuierlich zuzugeben, daß die vorgelegte Enzymmenge die zulaufende Saccharosemenge sofort umsetzen kann und so eine Anreicherung von Saccharose im Reaktionsgemisch, die zur unkontrollierten Bildung von hochmolekularem Dextran führen kann, vermieden wird. In jedem Fall sollte der Anteil der Saccharose an der Kohlehydrat-Trockensubstanz des Reaktionsgemisches im Gleichgewichtszustand der kontinuierlichen Umsetzung 25% nicht überschreiten.

Anstelle der gereinigten Dextransucrase kann auch das Gemisch aus dem Enzym und den es produzierenden Bakterien eingesetzt werden.

Die Aufbaureaktionen lassen sich z.B. wie folgt darstellen:

$$\text{Glucose} + n\ \text{Saccharose} \xrightarrow{\text{Enzym}} \text{iso-Malto-}(n+1)\text{-Saccharid} + n\ \text{Fructose} \qquad \text{I}$$

$$\text{Maltose} + n\ \text{Saccharose} \xrightarrow{\text{Enzym}} \text{Gluco-}(n+2)\text{-Saccharid} + n\ \text{Fructose} \qquad \text{II}$$

Hierin bedeutet n die Anzahl der Mole der eingesetzten Saccharose, deren Glucoseeinheiten zum Aufbau des Oligosaccharids dienen, während eine entsprechende Molzahl Fructose freigesetzt wird. Wenn n=9 ist, entsteht nach der Formel I die iso-Malto-Decaose (IM-10).

Die Aufbaureaktionen lassen sich erfindungsgemäß so steuern, daß Oligo- oder Polysaccharide des jeweils gewünschten Molekulargewichts erhalten werden. Unter den genannten Bedingungen der Temperatur und der Wasserstoffionenkonzentration hängt das bei dieser Aufbausynthese erreichte Molekulargewicht von der auf eine bestimmte Enzymaktivität in der vorgelegten Lösung bezogenen molaren Menge des Akzeptors und dem Molverhältnis der insgesamt zugeführten Saccharose zum Akzeptor ab.

Die Enzym-Aktivitätseinheit U (=Unit) ist die Menge der α(1→6)-D-Glucosyltransferase, die 1 μMol Saccharose pro Minute bei pH 5,2 und 298 K umsetzt. Wenn mehr Saccharose zugeführt wird, als die vorgelegte Enzym-Aktivität umsetzen kann, ist die Steuerung der Molekülgröße nicht mehr möglich.

Legt man als Enzymaktivität 1000 U zugrunde, so wird bei einem Saccharosezusatz von insgesamt 1000 mMol und 200—500 mMol Glucose das gewünschte Oligo-Saccharidgemisch mit einem mittleren Molekulargewicht von etwa 2000 bis 5000 erhalten.

Es ist also möglich, in wenigen Vorversuchen mit wechselnden molaren Mengen an Glucose innerhalb des aufgezeigten Bereichs bei vorgegebener Aktivität der α(1→6)-D-Glucosyltransferase (z.B. 1000 U) und einer konstanten Menge Saccharose (z.B. 1000 mMol), die Anlagerung der D-Glucopyranosyl-Gruppen der Saccharose an den Akzeptor so zu steuern, daß in hoher Ausbeute Fraktionen der jeweils gewünschten Oligo-bzw. Polysaccharide mit enger Molekulargewichtsverteilung synthetisiert werden können.

Man kann die gesamte erforderliche Menge der Glucose oder Maltose vorlegen oder unter Wahrung der übrigen Reaktionsbedingungen, insbesondere der Konzentrationsverhältnisse diese Saccharide in dem Maße ersetzen, wie sie als Akzeptor verbraucht werden. Es ist also möglich, die Synthesereaktion voll kontinuierlich durchzuführen.

Es ist ein unerwarteter Vorteil der erfindungsgemäßen Verfahrensweise, daß der Kohlenhydrat-Trockensubstanz-Gehalt des Reaktionsgemisches sehr hoch sein kann, indem er 30 bis 50%, insbesondere 40 bis 50%, beträgt.

Obgleich bei der erfindungsgemäßen enzymatischen Aufbausynthese steril gearbeitet wird, wie das z.B. für die Synthese von nativem Dextran üblich ist, können dem Reaktionsgemisch zur Vermeidung von unerwünschtem Hefewachstum Antimitotika (Mytosehemmer), wie schwefelige Säure in Mengen bis zu 1000 mg/kg, insbesondere 400 bis 600 mg/kg, zugesetzt werden.

Wenn für die Verwendung der Anteil der Mono- und Disaccharide im Reaktionsprodukt unerwünscht ist, werden deraus die Oligosaccharide durch Fällungsfraktionierung oder Chromatographie abgetrennt.

Dieses Produkt kann als Träger für Süßstoffe und als Ausgangsprodukt zur Herstellung von Eisendextran verwendet werden.

Gegenstand der Erfindung ist ferner ein Gemisch von Oligosacchariden mit bis zu 30, insbesondere 10 bis 20 Anhydroglucoseeinheiten und mehr Fructose als Glucose, wie es bei dem erfindungsgemäßen Verfahren anfällt. Dieses Gemisch eignet sich als körpergebendes Süßungsmittel.

Beispiel 1

In 16 l einer wässrigen Lösung des Enzyms Dextransucrase, die eine Aktivität von 5400 U/l hatte, wurden 7,3 kg kristalline Glucose bei 298 K gelöst. Der pH-Wert der Lösung betrug 5,4. Zu dieser Lösung wurden kontinuierlich 2,6 kg/h einer 40% Saccharoselösung mit einem pH-Wert von 5,4 gepumpt. Nach 48 h wurde die Zugabe der Saccharose beendet und das Enzym nach weiteren 2 Stunden durch Erwärmen des Reaktionsgemisches auf 70°C inaktiviert.

Aus einer Probe des Reaktionsgemisches wurden durch Gelchromatographie der Mono- und Disaccharidanteil abgetrennt und das Zahlenmittel Mn des Molekulargewichts der Oligosaccharidfraktion nach der Somogyi-Phosphat-Methode (Method in Carbohydrate Chemistry, Vol. I, (1962), S.384—386) bestimmt. Es betrug $M_n=2540$, was einem mittleren Polymerisationsgrad von 15,7 Anhydroglucoseeinheiten entspricht. Der Fruktoseanteil an der Kohlehydrattrockensubstanz betrug 45,0%, der Glucoseanteil 3,6%.

Beispiel 2
iso-Malto-Oligosaccharid-Gemische als Träger für Süßungsmittel

In 4,9 l der durch Chromatographie über eine mit einem stark sauren Kationenaustauscher in der $Ca^{++}$-Form gefüllten Säule erhaltenen iso-Malto-Oligosaccharid-Fraktion, mit einem Trockensubstanzgehalt von 17,2 Gew.% und einer mittleren Molmasse von 1250 g/Mol, wurden 7,1 g des Süßstoffes Acesulfam-K Hoechst® gelöst. Durch Trocknen der Lösung wurden 840 g eines weißen, in Wasser leicht löslichen Pulvers erhalten, das einen angenehmen süßen Geschmack hatte ("süßer" iso-Malto-Oligosaccharidträger).

| Unter Verwendung von | |
|---|---|
| tiefgefrorenen Erdbeeren | 1000 g |
| "süßem"-iso-Malto-Oligo-saccharidträger | 500 g |
| Geliermittel Opekta GP "Zwei zu Eins" | 25 g |

wurde eine Erdbeerkonfitüre gekocht. Die Koch-, Gelier- und Farbeigenschaften entsprachen denen einer mit 500 g Saccharose gekochten Erdbeerkonfitüre.

**Patentansprüche**

1. Verfahren zur Herstellung eines Gluco-oligosaccharid-Gemisches mit bis zu 30, insbesondere 10 bis 20 Anhydroglucoseeinheiten, dadurch gegekennzeichnet, daß man einer wässrigen Lösung eines aus D-Glucoseeinheiten aufgebauten Mono- oder Disaccharids, die mehr als 200 mMol des Saccharids pro 1000 U α(1→6)-D-Glucosyltransferase enthält, bei 265 bis 310 K und einem pH-Wert von 4,5 bis 8,0 eine wässrige Saccharoselösung derart zugibt, daß das Verhältnis der insgesamt zugefügten Saccharosemenge zu der Glucosemenge 2,0 bis 5,0 beträgt und der Anteil der Saccharose an der Kohlehydrat-Trockensubstanz des Reaktionsgemisches im Gleichgewichtszustand der kontinuierlichen Umsetzung 25% nicht überschreitet.

2. Verfahren nach Anspruch 1, dadurch gegekenn-

zeichnet, daß die wässrige Glucoselösung 400 bis 600 mMol Glucose pro 1000 U Enzym enthält.

3. Verfahren nach Anspruch 1 oder 2, dadurch gekennzeichnet, daß die Enzymreaktion bei 290 bis 300 K durchgeführt wird.

4. Verfahren nach einem der Ansprüche 1 bis 3, dadurch gekennzeichnet, daß der pH-Wert des Reaktionsgemisches 5 bis 6,5 beträgt.

5. Verfahren nach einem der Ansprüche 1 bis 4, dadurch gekennzeichnet, daß das Molverhältnis von Saccharose zu Glucose 3,0 bis 4,0 beträgt.

6. Verfahren nach einem der Ansprüche 1 bis 5, dadurch gekennzeichnet, daß man die Saccharoselösung kontinuierlich zugibt.

7. Verfahren nach einem der Ansprüche 1 bis 6, dadurch gekennzeichnet, daß die Saccharoselösung mit einer solchen Geschwindigkeit zugegeben wird, daß die Saccharose direkt von der α(1→6)-D-Glucosyltransferase umgesetzt wird.

8. Verfahren nach einem der Ansprüche 1 bis 7, dadurch gekennzeichnet, daß als aus D-Glucosyleinheiten aufgebautes Mono- oder Disaccharid Glucose eingesetzt wird.

9. Verfahren nach Anspruch 8, dadurch gekennzeichnet, daß die Glucose in Form eines Stärkehydrolysats mit hohem Dextrose-Äquivalent eingesetzt wird.

10. Verfahren nach einem der Ansprüche 1 bis 7, dadurch gekennzeichnet, daß als aus D-Glucosyleinheiten aufgebautes Mono- oder Disaccharid Maltose eingesetzt wird.

11. Verfahren nach einem der Ansprüche 1 bis 10, dadurch gekennzeichnet, daß der Kohlehydrattrockensubstanzgehalt des Reaktionsgemisches 30 bis 50%, insbesondere 40 bis 50%, beträgt.

12. Verfahren nach einem der Ansprüche 1 bis 10, dadurch gekennzeichnet, daß man als α(1→6)-D-Glucosyltransferase die vom Bakterium Leuconostoc mesenteroides, insbesondere dem Stamm B-512, ausgeschiedene Dextransucrase verwendet.

13. Verfahren nach einem der Ansprüche 1 bis 10, dadurch gekennzeichnet, daß man als α(1→6)-D-Glucosylatransferase die vom Bakterium Leuconostoc dextranicum ausgeschiedene Dextransucrase verwendet.

14. Verfahren nach einem der Ansprüche 1 bis 13, dadurch gekennzeichnet, daß man das aus D-Glucosyleinheiten aufgebaute Mono- oder Disaccharid kontinuierlich in dem Maße ersetzt, wie es als Akzeptor verbraucht wird.

15. Verfahren nach einem der Ansprüche 1 bis 14, dadurch gekennzeichnet, daß man dem Reaktionsgemisch zur Vermeidung von unerwünschtem Hefewachstum Antimitotika (Mytosehemmer) zusetzt.

16. Verfahren nach Anspruch 15, dadurch gekennzeichnet, daß man schwefelige Säure in Mengen bis zu 1000 mg/kg, vorzugsweise 400 bis 600 mg/kg, zusetzt.

17. Verfahren nach einem der Ansprüche 1 bis 16, dadurch gekennzeichnet, daß man aus dem Reaktionsprodukt das Gemisch der Oligosaccharide durch Fällungsfraktionierung oder Chromatographie abtrennt.

18. Verwendung des nach Anspruch 17 erhaltenen Gemisches als kalorienfreier Träger für Süßstoffe.

19. Verwendung des in einem Verfahren nach einem der Ansprüche 1 bis 17 erhaltenen Gemisches als körpergebendes Süßungsmittel.

**Revendications**

1. Procédé de production d'un mélange de glucooligosaccharides ayant jusqu'à 30, notamment 10 à 20 motifs anhydroglucose, caractérisé en ce qu'on ajoute à une solution aqueuse d'un monosaccharide ou d'un disaccharide constitué de motifs D-glucose, qui contient plus de 200 mmoles du saccharide pour 1000 U d'α(1→6)-D-glucosyltransférase, à 265—310 K et à une valeur de pH de 4,5 à 8,0, une solution aqueuse de saccharose de façon telle que le rapport de la quantité totale ajoutée de saccharose à la quantité de glucose ait une valeur de 2,0 à 5,0 et que la contribution du saccharose à la substance glucidique sèche du mélange réactionnel à l'état d'équilibre de la réaction continue ne dépasse pas 25%.

2. Procédé suivant la revendication 1, caractérisé en ce que la solution aqueuse de glucose contient 400 à 600 mmoles de glucose pour 1000 unités d'enzyme.

3. Procédé suivant la revendication 1 ou 2, caractérisé en ce que la réaction enzymatique est conduite à 290—300 K.

4. Procédé suivant l'une des revendications 1 à 3, caractérisé en ce que la valeur du pH du mélange réactionnel est de 5 à 6,5.

5. Procédé suivant l'une des revendications 1 à 4, caractérisé en ce que le rapport molaire du saccharose au glucose a une valeur de 3,0 à 4,0.

6. Procédé suivant l'une des revendications 1 à 5, caractérisé en ce qu'on ajoute en continu la solution de saccharose.

7. Procédé suivant l'une des revendications 1 à 6, caractérisé en ce que la solution de saccharose est ajoutée à une vitesse choisie de manière que le saccharose soit directement transformé par l'α(1→6)-D-glucosyltransférase.

8. Procédé suivant l'une des revendications 1 à 7, caractérisé en ce qu'on utilise le glucose comme monosaccharide ou disaccharide constitué de motifs D-glucosyle.

9. Procédé suivant la revendication 8, caractérisé en ce qu'on utilise le glucose sous forme d'un hydrolysat d'amidon à haut équivalent de dextrose.

10. Procédé suivant l'une des revendications 1 à 7, caractérisé en ce qu'on utilise le maltose comme monosaccharide ou disaccharide constitué de motifs D-glucosyle.

11. Procédé suivant l'une des revendications 1 à 10, caractérisé en ce que la teneur en substance glucidique sèche du mélange réactionnel est de 30 à 50%, notamment de 40 à 50%.

12. Procédé suivant l'une des revendications 1 à 10, caractérisé en ce qu'on utilise comme α(1→6)-D-glucosyltransférase, la dextranesucrase extraite de la bactérie Leuconostoc mesenteroides, appartenant notamment à la souche B-512.

13. Procédé suivant l'une des revendications 1 à 10, caractérisé en ce qu'on utilise comme α(1→6)-D-glucosyltransférase, la dextranesucrase extraite de la bactérie Leuconostoc dextranicum.

14. Procédé suivant l'une des revendications 1 à 13, caractérisé en ce que le monosaccharide ou le disaccharide constitué de motifs D-glucosyle est remplacé continuellement au fur et à mesure qu'il est consommé comme accepteur.

15. Procédé suivant l'une des revendications 1 à 14, caractérisé en ce qu'on ajoute au mélange réactionnel, des antimitotiques (inhibiteurs de mitose) pour éviter le développement indésirable de levures.

16. Procédé suivant la revendication 15, caractérisé en ce qu'on ajoute de l'acide sulfureux en quantités allant jusqu'à 1000 mg/kg, de préférence de 400 à 600 mg/kg.

17. Procédé suivant l'une des revendications 1 à 16, caractérisé en ce que le mélange d'oligosaccharides est séparé du produit réactionnel par fractionnement par précipitation, ou par chromatographie.

18. Utilisation du mélange obtenu selon la revendication 17 comme support dépourvu de calories pour substances sucrantes.

19. Utilisation du mélange obtenu dans un procédé selon l'une des revendications 1 à 17 comme édulcorant donnant du corps.

**Claims**

1. Method for preparing a gluco-oligosaccharide mixture with up to 30, in particular 10 to 20, anhydroglucose units, characterised in that an aqueous saccharose solution is added to an aqueous solution of a monosaccharide or disaccharide built up of D-glucose units which contains more than 200 mMol of the saccharide per 1000 U α(1→6)-D-glucosyltransferase at 265 to 310 K and a pH value of 4.5 to 8.0 in such a manner that the ratio of the total added quantity of saccharose to the quantity of glucose is 2.0 to 5.0 and the proportion of the saccharose in the carbohydrate dry substance of the reaction mixture in the equilibrium state of the continuous reaction does not exceed 25%.

2. Method according to Claim 1, characterised in that the aqueous glucose solution contains 400 to 600 mMol glucose per 1000 U enzyme.

3. Method according to Claim 1 or 2, characterised in that the enzyme reaction is carried out at 290 to 300 K.

4. Method according to one of Claims 1 to 3, characterised in that the pH value of the reaction mixture is 5 to 6.5.

5. Method according to one of Claims 1 to 4, characterised in that the molar ratio of saccharose to glucose is 3.0 to 4.0.

6. Method according to one of Claims 1 to 5, characterised in that the saccharose solution is added continuously.

7. Method according to one of Claims 1 to 6, characterised in that the saccharose solution is added at such a rate that the saccharose is reacted directly from the α(1→6)-D-glucosyltransferase.

8. Method according to one of Claims 1 to 7, characterised in that glucose is used as the monosaccharide or disaccharide composed of D-glucosyl units.

9. Method according to Claim 8, characterised in that the glucose is used in the form of a starch hydrolysate with a high dextrose equivalent.

10. Method according to one of Claims 1 to 7, characterised in that maltose is used as the monosaccharide or disaccharide composed of D-glucosyl units.

11. Method according to one of Claims 1 to 10, characterised in that the carbohydrate dry substance content of the reaction mixture is 30 to 50%, in particular 40 to 50%.

12. Method according to one of Claims 1 to 10, characterised in that the dextransucrase separated from the bacterium leuconostoc mesenteroides, in particular the B-512 strain, is used as the α(1→6)-D-glucosyltransferase.

13. Method according to one of Claims 1 to 10, characterised in that the dextransucrase separated from the bacterium leuconostoc dextranicum is used as the α(1→6)-D-glucosyltransferase.

14. Method according to one of Claims 1 to 13, characterised in that the monosaccharide or disaccharide composed of D-glucosyl units is replaced continually to the extent by which it is consumed as an acceptor.

15. Method according to one of Claims 1 to 14, characterised in that anti-mitotic agents (mitosis inhibitors) are added to the reaction mixture in order to eliminate unwanted growth of yeasts.

16. Method according to Claim 15, characterised in that sulphurous acid is added in quantities of up to 1000 mg/kg, preferably 400 to 600 mg/kg.

17. Method according to one of Claims 1 to 16, characterised in that the mixture of oligosaccharides is separated off from the reaction product by fractionated precipitation or chromatography.

18. Use of the mixture obtained according to Claim 17 as a calorie-free support for sweeteners.

19. Use of the mixture obtained in a process according to one of Claims 1 to 17 as a body-giving sweetening agent.